(19) Europäisches Patentamt
European Patent Office
Office européen des brevets

(11) EP 1 353 634 B1

(12) # FASCICULE DE BREVET EUROPEEN

(45) Date de publication et mention de la délivrance du brevet:
**24.01.2007 Bulletin 2007/04**

(21) Numéro de dépôt: **02711942.9**

(22) Date de dépôt: **11.01.2002**

(51) Int Cl.:
***A61K 8/73*** *(2006.01)* ***A61Q 5/12*** *(2006.01)*
***A61K 8/81*** *(2006.01)* ***A61K 8/895*** *(2006.01)*

(86) Numéro de dépôt international:
**PCT/FR2002/000108**

(87) Numéro de publication internationale:
**WO 2002/055036 (18.07.2002 Gazette 2002/29)**

(54) **COMPOSITIONS COSMETIQUES CONTENANT UN FRUCTANE ET POLYMERE CATIONIQUE ET LEURS UTILISATIONS**

KOSMETISCHE ZUSAMMENSETZUNGEN DIE EIN FRUCTAN UND EIN KATIONISCHES POLYMER ENTHALTEN UND DEREN VERWENDUNG

COSMETIC COMPOSITIONS CONTAINING A FRUCTAN AND A CATIONIC POLYMER AND THEIR USES

(84) Etats contractants désignés:
**AT BE CH CY DE DK ES FI FR GB GR IE IT LI LU MC NL PT SE TR**

(30) Priorité: **12.01.2001 FR 0100410**

(43) Date de publication de la demande:
**22.10.2003 Bulletin 2003/43**

(73) Titulaire: **L'ORÉAL**
**75008 Paris (FR)**

(72) Inventeurs:
- **FACK, Géraldine**
  **F-92300 Levallois (FR)**
- **POURILLE-GRETHEN, Chrystel**
  **F-92110 Clichy (FR)**
- **RESTLE, Serge**
  **F-95390 Saint-Prix (FR)**

(74) Mandataire: **Le Blainvaux Bellegarde, Françoise**
**L'OREAL - D.I.P.I.**
**25-29 Quai Aulagnier**
**92600 Asnières (FR)**

(56) Documents cités:
**EP-A- 1 174 118**
**WO-A-98/05793**
**WO-A-98/14482**
**DE-A- 10 004 644**
**FR-A- 2 795 953**

Remarques:
Le dossier contient des informations techniques présentées postérieurement au dépôt de la demande et ne figurant pas dans le présent fascicule.

EP 1 353 634 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention concerne de nouvelles compositions cosmétiques comprenant dans un milieu cosmétiquement acceptable au moins un polymère cationique et au moins un fructane.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératinique et en particulier des cheveux, des associations de polymères à caractères épaississants. Parmi celles-ci, ont été décrites les associations de polysaccharides tels que l'inuline et de terpolymères acrylique à motif uréthane.
De telles associations présentent cependant des inconvénients tels que des problèmes de rinçabilité, des problèmes de stabilité à pH acide, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques pour faciliter le démêlage des cheveux et pour leur communiquer douceur et souplesse. L'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

WO9814482 décrit des compositions cosmétiques comprenant un fructane cationique, comportant une fonction amine.
EP 1025839 et GB2220216 décrivent des compositions détergentes contenant au moins un polymère cationique, ces compositions ne contiennent pas de fructane.

En résumé, il s'avère que les compositions cosmétiques actuelles contenant des polymères cationiques, ne donnent pas complètement satisfaction.

La demanderesse a maintenant découvert que l'association d'un fructane avec des polymères cationiques permet de remédier à ces inconvénients.

Ainsi, à la suite d'importantes recherches menées sur la question, il a maintenant été trouvé par la Demanderesse qu'en introduisant un fructane dans les compositions en particulier capillaires de l'art antérieur à base de polymères cationiques, il est possible de limiter, voire supprimer, les problèmes évoqués ci-dessus.

De plus, cette association apporte une texture fondante aux compositions cosmétiques, c'est à dire qui disparaît rapidement dans la chevelure. Les cheveux traités avec cette composition ont un toucher doux et sans résidus.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un polymère cationique et au moins un fructane,
le fructane ne comportant pas de fonction amine

Un autre objet de l'invention concerne l'utilisation d'un fructane dans, ou pour la fabrication d'une composition cosmétique comprenant un polymère cationique.

Les différents objets de l'invention vont maintenant être détaillés. L'ensemble des significations et définitions des composés utilisés dans la présente invention données ci-dessous sont valables pour l'ensemble des objets de l'invention.

Les fructanes ou fructosanes sont des oligosaccharides ou des polysaccharides comprenant un enchaînement d'unités anhydrofructose éventuellement associé à un plusieurs résidus saccharidiques différents du fructose. Les fructanes peuvent être linéaires ou ramifiés. Les fructanes peuvent être des produits obtenus directement à partir d'une source végétale ou microbienne ou bien des produits dont la longueur de chaîne a été modifiée (augmentée ou réduite) par fractionnement, synthèse ou hydrolyse en particulier enzymatique. Les fructanes ont généralement un degré de polymérisation de 2 à environ 1000 et de préférence de 3 à environ 60.
On distingue 3 groupes de fructanes. Le premier groupe correspond à des produits dont les unités fructose sont pour la plupart liées par des liaisons β-2-1.Ce sont des fructanes essentiellement linéaires tes que les inulines.
Le second groupe correspond également à des fructoses linéaires mais les unités fructose sont essentiellement liées par des liaisons β-2-6. Ces produits sont des levanes.
Le troisième groupe correspond à des frucanes mixtes, c'est à dire ayant des enchainements β-2-6 et β-2-1. Ce sont des fructanes essentiellement ramifiés tes que les graminanes.

Les fructanes préférés sont les inulines. L'inuline peut être obtenue par exemple à partir de chicorée, de dahlia ou de topinambours.

Les fructanes selon l'invention ne comportent pas de groupement amine.

Selon l'invention, on désigne par groupement amine tout groupement comportant au moins une amine primaire,

secondaire, tertiaire ou un groupement ammonium quaternaire.

Les fructanes peuvent aussi ne pas être modifiés par une oxyde d'alkylène et/ou par un agent d'alkylation.

Les fructanes n'étant pas modifiés par un oxyde d'alkylène et/ou par un agent d'alkylation ne contiennent donc pas de groupement oxyalkyléné et/ou de groupement alkyle.

Le fructane est utilisée de préférence en une quantité comprise entre 0,01 et 20% en poids par rapport au poids total de la composition. Plus préférentiellement, cette quantité est comprise entre 0,05 et 15% en poids par rapport au poids total de la composition et encore plus préférentiellement entre 0,1 et 10% en poids.

Les polymères cationiques utilisables conformément à la présente invention peuvent être choisis parmi tous ceux déjà connus en soi comme améliorant les propriétés cosmétiques des cheveux, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire moyenne en nombre comprise entre 500 et $5.10^6$ environ, et de préférence comprise entre $10^3$ et $3.10^6$ environ.

Parmi les polymères cationiques, on peut citer plus particulièrement les polymères du type polyamine, polyaminoamide et polyammonium quaternaire. Ce sont des produits connus.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :

(1) les homopolymères ou copolymères dérivés d'esters ou d'amides acryliques ou méthacryliques et comportant au moins un des motifs de formules suivantes:

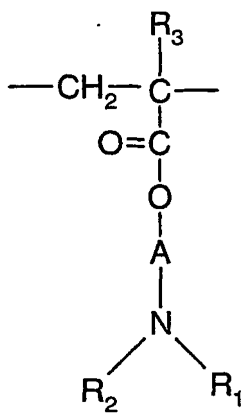
,
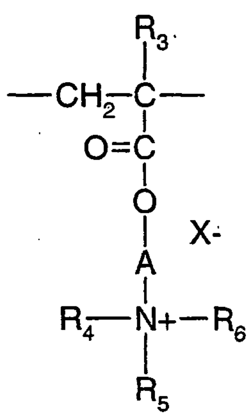

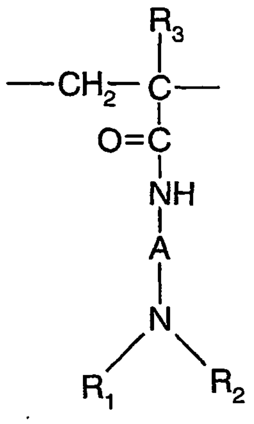

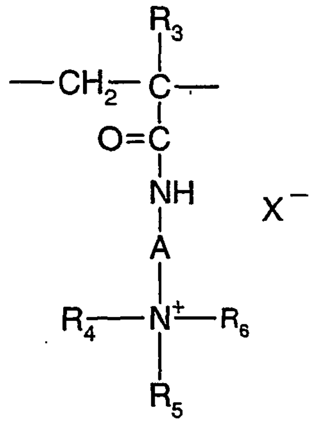

dans lesquelles :

$R_3$, identiques ou différents, désignent un atome d'hydrogène ou un radical $CH_3$ ;
A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
$R_4$, $R_5$, $R_6$, identiques ou différents, représentent un groupe alkyle ayant de 1 à 18 atomes de carbone ou un radical benzyle et de préférence un groupe alkyle ayant de 1 à 6 atomes de carbone ;
$R_1$ et $R_2$, identiques ou différents, représentent hydrogène ou un groupe alkyle ayant de 1 à 6 atomes de carbone et de préférence méthyle ou éthyle;
X désigne un anion dérivé d'un acide minéral ou organique tel que un anion méthosulfate ou un halogénure tel que chlorure ou bromure.

Les copolymères de la famille (1) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
Ainsi, parmi ces copolymères de la famille (1), on peut citer :

- les copolymères d'acrylamide et de diméthylaminoéthyl méthacrylate quatemisé au sulfate de diméthyle ou avec un halogénure de diméthyle tels que celui vendu sous la dénomination HERCOFLOC par la société HERCULES,
- les copolymères d'acrylamide et de chlorure de méthacryloyloxyéthyltriméthylammonium décrit par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthylammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP.
- et les copolymères vinylpyrrolidone / méthacrylamide de diméthylaminopropyle quaternisé tel que le produit vendu sous la dénomination "GAFQUAT HS 100" par la société ISP.

(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis

dans le dictionnaire CTFA comme des ammonium quaternaires d'hydroxyéthylcellulose ayant réagi avec un époxyde substitué par un groupement triméthylammonium.

(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
Les produits commercialisés répondant à cette définition sont plus particulièrement les produits vendus sous la dénomination "Celquat L 200" et "Celquat H 100" par la Société National Starch.

(4) Les polysaccharides cationiques décrits plus particulièrement dans les brevets US 3 589 578 et 4 031 307 tels que les gommes de guar contenant des groupements cationiques trialkylammonium. On utilise par exemple des gommes de guar modifiées par un sel (par ex. chlorure) de 2,3-époxypropyl triméthylammonium.
De tels produits sont commercialisés notamment sous les dénominations commerciales de JAGUAR C13 S, JAGUAR C 15, JAGUAR C 17 ou JAGUAR C162 par la société MEYHALL.

(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;

(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;

(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.

(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.

(9) les cyclopolymères d'alkyl diallyl amine ou de dialkyl diallyl ammonium tels que les homopolymères ou copolymères comportant comme constituant principal de la chaîne des motifs répondant aux formules (VI) ou (VI') :

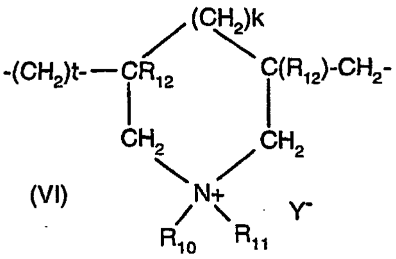

(VI)

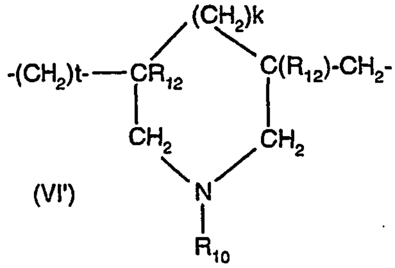

(VI')

formules dans lesquelles k et t sont égaux à 0 ou 1, la somme k + t étant égale à 1 ; $R_{12}$ désigne un atome d'hydrogène ou un radical méthyle ; $R_{10}$ et $R_{11}$, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou $R_{10}$ et $R_{11}$ peuvent désigner conjointement avec l'atome d'azote auquel ils sont rattachés, des groupement hétérocycliques, tels que pipéridinyle ou morpholinyle ; Y est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406. $R_{10}$ et $R_{11}$, indépendamment l'un de l'autre, désignent de préférence un groupement alkyle ayant de 1 à 4 atomes de carbone.

Parmi les polymères définis ci-dessus, on peut citer plus particulièrement l'homopolymère de chlorure de diméthyldiallylammonium vendu sous la dénomination "Merquat 100" par la société Calgon (et ses homologues de faibles masses moléculaires moyenne en poids) et les copolymères de chlorure de diallyldiméthylammonium et d'acrylamide commercialisés sous la dénomination "MERQUAT 550".

(10) le polymère de diammonium quaternaire contenant des motifs récurrents répondant à la formule :

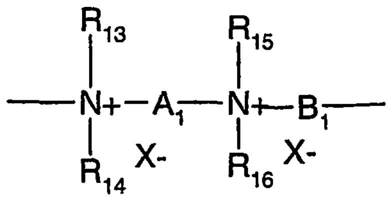

(VII)

formule (VII) dans laquelle :

$R_{13}$, $R_{14}$, $R_{15}$ et $R_{16}$, identiques ou différents, représentent des radicaux aliphatiques, alicycliques, ou arylaliphatiques contenant de 1 à 20 atomes de carbone ou des radicaux hydroxyalkylaliphatiques inférieurs, ou bien $R_{13}$, $R_{14}$, $R_{15}$ et $R_{16}$, ensemble ou séparément, constituent avec les atomes d'azote auxquels ils sont rattachés des hétérocycles contenant éventuellement un second hétéroatome autre que l'azote ou bien $R_{13}$, $R_{14}$, $R_{15}$, et $R_{16}$ représentent un radical alkyle en C1-C6 linéaire ou ramifié substitué par un groupement nitrile, ester, acyle, amide ou -CO-O-$R_{17}$-D ou -CO-NH-$R_{17}$-D où $R_{17}$ est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et X- désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène

ou hydroxyalkylène linéaire ou ramifié, saturé ou insaturé, B1 peut également désigner un groupement (CH2)n-CO-D-OC-(CH2)n-
dans lequel D désigne :

a) un reste de glycol de formule : -O-Z-O-, où Z désigne un radical hydrocarboné linéaire ou ramifié ou un groupement répondant à l'une des formules suivantes :

-(CH2-CH2-O)x-CH2-CH2-

-[CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent

-CH2-CH2-S-S-CH2-CH2- ;

d) un groupement uréylène de formule : -NH-CO-NH- ;

De préférence, $X^-$ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
On peut utiliser plus particulièrement les polymères qui sont constitués de motifs récurrents répondant à la formule :

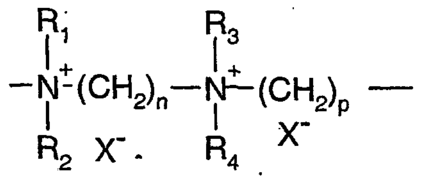 (a)

dans laquelle $R_1$, $R_2$, $R_3$ et $R_4$, identiques ou différents, désignent un radical alkyle ou hydroxyalkyle ayant de 1 à 4 atomes de carbone environ, n et p sont des nombres entiers variant de 2 à 20 environ et, $X^-$ est un anion dérivé d'un acide minéral ou organique.
Un composé de formule (a) particulièrement préféré est celui pour lequel $R_1$, $R_2$, $R_3$ et $R_4$, représentent un radical méthyle et n = 3, p = 6 et X = Cl, dénommé Hexadimethrine chloride selon la nomenclature INCI (CTFA).

(11) les polymères de polyammonium quaternaires constitués de motifs de formule (VIII):

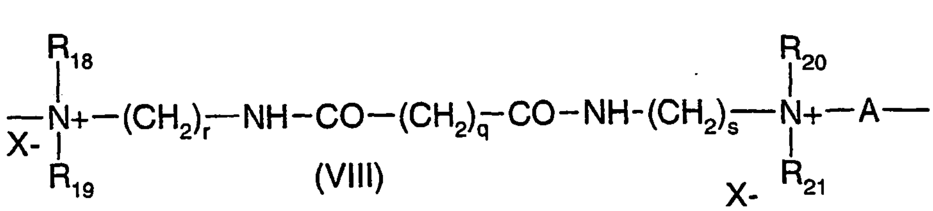
(VIII)

formule dans laquelle :

$R_{18}$, $R_{19}$, $R_{20}$ et $R_{21}$, identiques ou différents, représentent un atome d'hydrogène ou un radical méthyle, éthyle, propyle, β-hydroxyéthyle, β-hydroxypropyle ou $-CH_2CH_2(OCH_2CH_2)_pOH$,
où p est égal à 0 ou à un nombre entier compris entre 1 et 6, sous réserve que $R_{18}$, $R_{19}$, $R_{20}$ et $R_{21}$ ne représentent pas simultanément un atome d'hydrogène,
r et s, identiques ou différents, sont des nombres entiers compris entre 1 et 6,

q est égal à 0 ou à un nombre entier compris entre 1 et 34,
X- désigne un anion tel qu'un halogènure,
A désigne un radical d'un dihalogénure ou représente de préférence $-CH_2-CH_2-O-CH_2-CH_2-$.

De tels composés sont notamment décrits dans la demande de brevet EP-A-122 324.
On peut par exemple citer parmi ceux-ci, les produits "Mirapol® A 15", "Mirapol® AD1", "Mirapol® AZ1 " et "Mirapol® 175" vendus par la société Miranol.

(12) Les polymères quaternaires de vinylpyrrolidone et de vinylimidazole tels que par exemple les produits commercialisés sous les dénominations Luviquat® FC 905, FC 550 et FC 370 par la société B.A.S.F.

(13) Les polyamines comme le Polyquart® H vendu par HENKEL, référencé sous le nom de « POLYETHYLENEGLYCOL (15) TALLOW POLYAMINE » dans le dictionnaire CTFA.

(14) Les polymères réticulés de sels de méthacryloyloxyalkyl($C_1$-$C_4$) trialkyl($C_1$-$C_4$)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE® SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE® SC 95 » et « SALCARE® SC 96 » par la Société ALLIED COLLOIDS.

D'autres polymères cationiques utilisables dans le cadre de l'invention sont des protéines cationiques ou des hydrolysats de protéines cationiques, des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.
Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les cyclopolymères cationiques, en particulier les homopolymères ou copolymères de chlorure de diméthyldiallylammonium, vendus sous les dénominations « MERQUAT 100 », « MERQUAT 550 » et « MERQUAT S » par la société CALGON, les polymères quaternaires de vinylpyrrolidone et de vinylimidazole, les homopolymères ou copolymères réticulés de sels de méthacryloyloxyalkyl($C_1$-$C_4$) trialkyl($C_1$-$C_4$) ammonium et leurs mélanges.
Selon l'invention, le ou les polymères cationiques peuvent représenter de 0,001 % à 20 % en poids, de préférence de 0,01 % à 10% en poids et plus particulièrement de 0,1 à 5% en poids par rapport au poids total de la composition finale.
Selon un mode de réalisation particulièrement préféré, les compositions selon l'invention comprennent en outre au moins une silicone ou un autre agent bénéfique pour les cheveux tels que notamment les esters d'acides carboxyliques en C1-C30 et de d'alcools mono ou polyhydroxylés en C1-C30, les huiles végétales, animales, minérales ou de synthèse, les cires, les céramides, les pseudocéramides.
Les silicones utilisables conformément à l'invention sont en particulier des polyorganosiloxanes insolubles dans la composition et peuvent se présenter sous forme d'huiles, de cires, de résines ou de gommes.
Les organopolysiloxanes sont définis plus en détail dans l'ouvrage de Walter NOLL "Chemistry and Technology of Silicones" (1968) Academie Press. Elles peuvent être volatiles ou non volatiles.
Lorsqu'elles sont volatiles, les silicones sont plus particulièrement choisies parmi celles possédant un point d'ébullition compris entre 60° C et 260° C, et plus particulièrement encore parmi :

(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
On peut également citer les cyclocopolymères du type diméthylsiloxanes/méthylakylsiloxane, tel que la "SILICONE

VOLATILE FZ 3109" commercialisée par la société UNION CARBIDE, de structure chimique :

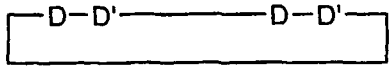

avec D : 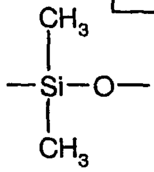 avec D' : 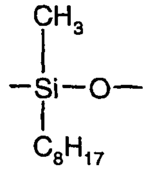

On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à $5.10^{-6}m^2/s$ à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.
Ces silicones sont plus particulièrement choisies parmi les polyalkylsiloxanes parmi lesquels on peut citer principalement les polydiméthylsiloxanes à groupements terminaux triméthylsilyle ayant une viscosité de $5.10^{-6}$ à 2,5 $m^2/s$ à 25°C et de préférence $1.10^{-5}$ à 1 $m^2/s$. La viscosité des silicones est par exemple mesurée à 25°C selon la norme ASTM 445 Appendice C.
Parmi ces polyalkylsiloxanes, on peut citer à titre non limitatif les produits commerciaux suivants :

- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.
Dans cette classe de polyalkylsiloxanes, on peut également citer les produits commercialisés sous les dénominations "ABIL WAX 9800 et 9801" par la société GOLDSCHMIDT qui sont des polyalkyl ($C_1$-$C_{20}$) siloxanes.
Les polyalkylarylsiloxanes sont particulièrement choisis parmi les polydiméthyl méthylphénylsiloxanes, les polydiméthyl diphénylsiloxanes linéaires et/ou ramifiés de viscosité de $1.10^{-5}$ à $5.10^{-2}m^2/s$ à 25°C.
Parmi ces polyalkylarylsiloxanes on peut citer à titre d'exemple les produits commercialisés sous les dénominations suivantes :

- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1 023, SF 1154, SF 1250, SF 1265.

Les gommes de silicone utilisables conformément à l'invention sont notamment des polydiorganosiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :

- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxane/diphénylsiloxane/méthylvinylsiloxane.

Des produits plus particulièrement utilisables conformément à l'invention sont des mélanges tels que :

- les mélanges formés à partir d'un polydiméthylsiloxane hydroxylé en bout de chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 $m^2/s$ et d'une huile SF 96 d'une viscosité de $5.10^{-6}{}_{M}{}^{2}/_{S}$. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :

$R_2SiO_{2/2}$, $R_3SiO_{1/2}$, $RSiO_{3/2}$ et $SiO_{4/2}$ dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en $C_1$-$C_4$, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :

- des groupements polyéthylèneoxy et/ou polypropylèneoxy comportant éventuellement des groupements alkyle en $C_6$-$C_{24}$ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl ($C_{12}$) méthicone copolyol commercialisée par la société DOW CORNING sous la dénomination Q2 5200 ;

- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en $C_1$-$C_4$ ;

- des groupements thiols comme les produits commercialisés sous les dénominations "GP 72 A" et "GP 71" de GENESEE ;

- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;

- des groupements hydroxylés comme les polyorganosiloxanes à fonction hydroxyalkyle décrits dans la demande de brevet français FR-A-85 16334 répondant à la formule (IX) :

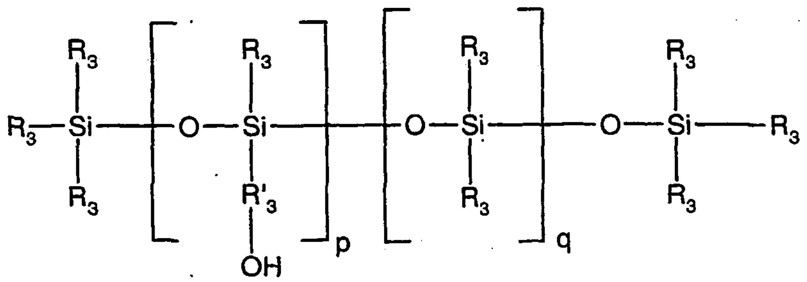

(IX)

dans laquelle les radicaux $R_3$ identiques ou différents sont choisis parmi les radicaux méthyle et phényle ; au moins 60 % en mole des radicaux $R_3$ désignant méthyle ; le radical $R'_3$ est un chaînon alkylène divalent hydrocarboné en $C_2$-$C_{18}$ ; p est compris entre 1 et 30 inclus ; q est compris entre 1 et 150 inclus;

- des groupements acyloxyalkyle tels que par exemple les polyorganosiloxanes décrits dans le brevet US-A-4957732 et répondant à la formule (X) :

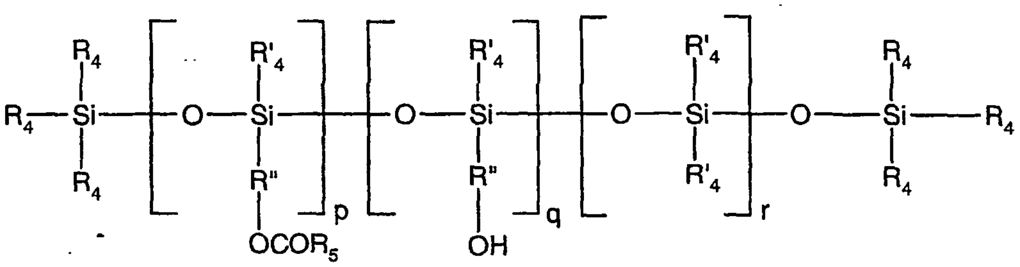

(X)

dans laquelle :

$R_4$ désigne un groupement méthyle, phényle, -$OCOR_5$, hydroxyle, un seul des radicaux $R_4$ par atome de silicium pouvant être OH ;
$R'_4$ désigne méthyle, phényle ; au moins 60 % en proportion molaire de l'ensemble des radicaux $R_4$ et $R'_4$ désignant méthyle ;
$R_5$ désigne alkyle ou alcényle en $C_8$-$C_{20}$ ;
R" désigne un radical alkylène hydrocarboné divalent, linéaire ou ramifié en $C_2$-$C_{18}$;
r est compris entre 1 et 120 inclus ;
p est compris entre 1 et 30 ;
q est égal à 0 ou est inférieur à 0,5 p, p + q étant compris entre 1 et 30 ; les polyorganosiloxanes de formule (VI) peuvent contenir des groupements :

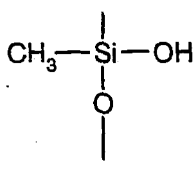

dans des proportions ne dépassant pas 15 % de la somme p + q + r.

- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations "ABIL S201 et "ABIL S255".

- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412704, EP-A-412707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.
De tels polymères sont par exemple les copolymères susceptibles d'être obtenus par polymérisation radicalaire à partir du mélange de monomères constitué par :

a) 50 à 90% en poids d'acrylate de tertiobutyle ;
b) 0 à 40% en poids d'acide acrylique ;
c) 5 à 40% en poids de macromère siliconé de formule :

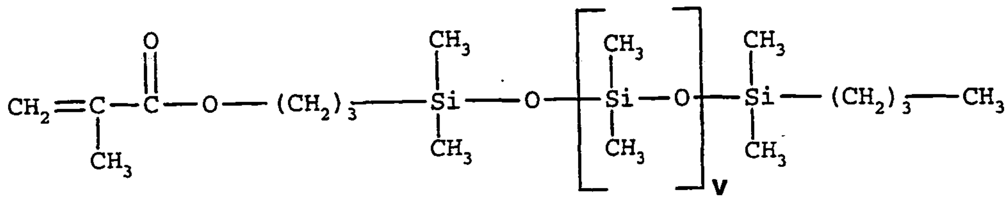

avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.
Selon l'invention, toutes les silicones peuvent également être utilisées sous forme d'émulsions, de nanoémulsions ou de micrémulsions.
Les polyorganosiloxanes particulièrement préférés conformément à l'invention sont :

- les silicones non volatiles choisies dans la famille des polyalkylsiloxanes à groupements terminaux triméthylsilyle telles que les huiles ayant une viscosité comprise entre 0,2 et 2,5 $m^2/s$ à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;

- la résine d'organopolysiloxane commercialisée sous la dénomination DOW CORNING 593 ;

- les polysiloxanes à groupements aminés tels que les amodiméthicones ou les triméthylsilylamodiméthicone ;

Selon l'invention, les silicones additionnelles ou les autres agents bénéfiques additionnels peuvent représenter de 0,001 % à 20 % en poids, de préférence de 0,01 % à 10% en poids et plus particulièrement de 0,1 à 5% en poids par rapport au poids total de la composition finale.
Les compositions de l'invention contiennent en outre avantageusement au moins un agent tensioactif qui est généralement présent en une quantité comprise entre 0,05% et 50% en poids environ, de préférence entre 0,1 % et 40% et encore plus préférentiellement entre 0,55% et 30%, par rapport au poids total de la composition.
Cet agent tensioactif peut être choisi parmi les agents tensioactifs anioniques, amphotères, non-ioniques, cationiques ou leurs mélanges.
Les tensioactifs convenant à la mise en oeuvre de la présente invention sont notamment les suivants :

(i) Tensioactif(s) anionique(s) :

Leur nature ne revêt pas, dans le cadre de la présente invention, de caractère véritablement critique. Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, $\alpha$-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl ($C_6$-$C_{24}$) éther carboxyliques polyoxyalkylénés, les acides alkyl($C_6$-$C_{24}$)aryl éther carboxyliques polyoxyalkylénés ,les acides alkyl($C_6$-$C_{24}$) amido éther carboxyliques polyoxyalkylénés et leurs sels, en particulier ceux comportant de 2 à 50 groupements oxyde d'éthylène, et leurs mélanges.
Parmi les tensioactifs anioniques, on préfère utiliser selon l'invention les sels d'alkylsulfates et d'alkyléthersufates et leurs mélanges.

(ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl ($C_{10}$ - $C_{14}$) amines ou les oxydes de N-acylaminopropylmorpholine. On notera que les alkylpolyglycosides constituent des tensioactifs non-ioniques rentrant particulièrement bien dans le cadre de la présente invention.

(iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl ($C_8$-$C_{20}$) bétaïnes, les sulfobétaïnes, les alkyl ($C_8$-$C_{20}$) amidoalkyl ($C_1$-$C_6$) bétaïnes ou les alkyl ($C_8$-$C_{20}$) amidoalkyl ($C_1$-$C_6$) sulfobétaïnes.
Parmi les dérivés d'amines, on peut citer les produits commercialisés sous les dénomination MIRANOL, tels que décrits dans les brevets US-2 528 378 et US-2 781 354 et de structures :

$$R_2\text{-}CONHCH_2CH_2\text{-}N(R_3)(R_4)(CH_2COO\text{-}) \qquad (2)$$

dans laquelle : $R_2$ désigne un radical alkyle dérivé d'un acide $R_2$-COOH présent dans l'huile de coprah hydrolysée, un radical heptyle, nonyle ou undécyle, $R_3$ désigne un groupement bêta-hydroxyéthyle et $R_4$ un groupement carboxyméthyle ;
et

$$R_5\text{-}CONHCH_2CH_2\text{-}N(B)(C) \qquad (3)$$

dans laquelle :

B représente $-CH_2CH_2OX'$, C représente $-(CH_2)_z$ -Y', avec z = 1 ou 2,
X' désigne le groupement $-CH_2CH_2$-COOH ou un atome d'hydrogène
Y' désigne -COOH ou le radical $-CH_2$ - CHOH - SO3H
$R_5$ désigne un radical alkyle d'un acide $R_9$ -COOH présent dans l'huile de coprah ou dans l'huile de lin hydrolysée, un radical alkyle, notamment en $C_7$, $C_9$, $C_{11}$ ou $C_{13}$, un radical alkyle en $C_{17}$ et sa forme iso, un radical $C_{17}$ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHONE POULENC.

(iv) Les tensioactifs cationiques peuvent être choisis parmi :

A) les sels d'ammonium quaternaires de la formule générale (IV) suivante :

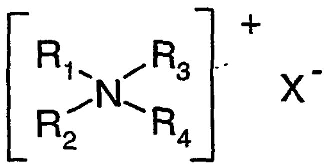

(IV)

dans laquelle X est un anion choisi dans le groupe des halogénures (chlorure, bromure ou iodure) ou alkyl($C_2$-$C_6$) sulfates plus particulièrement méthylsulfate, des phosphates, des alkyl-ou-alkylarylsulfonates, des anions dérivés d'acide organique tel que l'acétate ou le lactate.
et

a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
R4 désigne un radical alkyle, linéaire ou ramifié, comportant de 16 à 30 atomes de carbone.
De préférence le tensioactif cationique est un sel (par exemple chlorure) de béhényl triméthyl ammonium.

b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone ;
R3 et R4, identiques ou différents, désignent un radical alkyle, linéaire ou ramifié, comportant de 12 à 30 atomes de carbone, ledit radical comprenant au moins une fonction ester ou amide.
R3 et R4 sont notamment choisis parmi les radicaux alkyl($C_{12}$-$C_{22}$)amido alkyle($C_2$-$C_6$), alkyl($C_{12}$-$C_{22}$)acétate ;
De préférence le tensioactif cationique est un sel (par exemple chlorure) de stéaramidopropyl diméthyl (myristylacétate) ammonium.

B) - les sels d'ammonium quaternaire de l'imidazolinium, comme par exemple celui de formule (V) suivante :

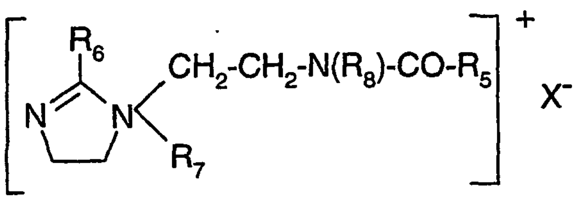

(V)

dans laquelle $R_5$ représente un radical alcényle ou alkyle comportant de 8 à 30 atomes de carbone par exemple dérivés des acides gras du suif, $R_6$ représente un atome d'hydrogène, un radical alkyle en $C_1$-$C_4$ ou un radical alcényle ou alkyle comportant de 8 à 30 atomes de carbone, $R_7$ représente un radical alkyle en Ci-$C_4$ , $R_8$ représente un atome d'hydrogène, un radical alkyle en $C_1$-$C_4$, X est un anion choisi dans le groupe des halogénures, phosphates, acétates, lactates, alkylsulfates, alkyl-ou-alkylarylsulfonates. De préférence, $R_5$ et $R_6$ désignent un mélange de radicaux alcényle ou alkyle comportant de 12 à 21 atomes de carbone par exemple dérivés des acides gras du suif, $R_7$ désigne méthyle, $R_8$ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,

C) - les sels de diammonium quaternaire de formule (VI) :

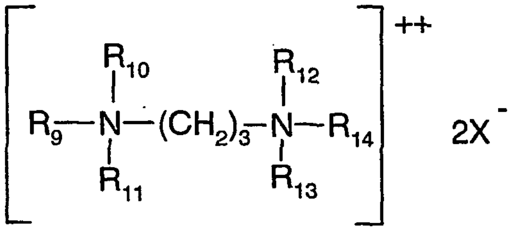

(VI)

dans laquelle $R_9$ désigne un radical aliphatique comportant environ de 16 à 30 atomes de carbone, $R_{10}$, $R_{11}$, $R_{12}$, $R_{13}$ et $R_{14}$ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.

D) - les sels d'ammonium quaternaire contenant au moins une fonction ester de formule (VII) suivante :

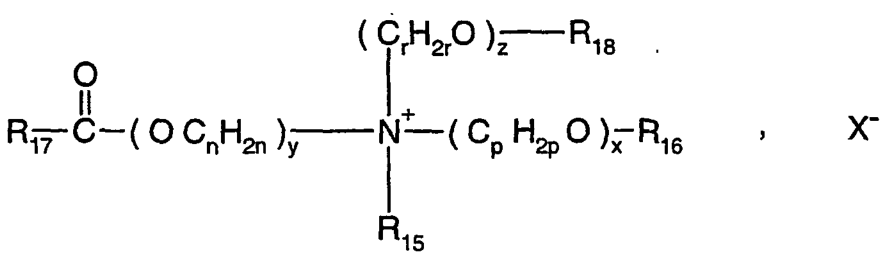

(VII)

dans laquelle :

- $R_{15}$ est choisi parmi les radicaux alkyles en $C_1$-$C_6$ et les radicaux hydroxyalkyles ou dihydroxyalkyles en $C_1$-$C_6$ ;
- $R_{16}$ est choisi parmi :
  - le radical

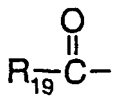

- les radicaux $R_{20}$ hydrocarbonés en $C_1$-$C_{22}$ linéaires ou ramifiés, saturés ou insaturés,
- l'atome d'hydrogène,

- $R_{18}$ est choisi parmi :

  - le radical

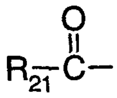

  - les radicaux $R_{22}$ hydrocarbonés en $C_1$-$C_6$ linéaires ou ramifiés, saturés ou insaturés,
  - l'atome d'hydrogène,

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;

sous réserve que la somme x + y + z vaut de 1 à 15 , que lorsque x vaut 0 alors $R_{16}$ désigne $R_{20}$ et que lorsque z vaut 0 alors $R_{18}$ désigne $R_{22}$.

On utilise plus particulièrement les sels d'ammonium de formule (VII) dans laquelle :

- $R_{15}$ désigne un radical méthyle ou éthyle,
- x et y sont égaux à 1 ;
- z est égal à 0 ou 1 ;
- n, p et r sont égaux à 2 ;
- $R_{16}$ est choisi parmi :

  - le radical

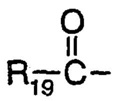

  - les radicaux méthyle, éthyle ou hydrocarbonés en $C_{14}$-$C_{22}$
  - l'atome d'hydrogène ;

- $R_{17}$, $R_{19}$ et $R_{21}$, identiques ou différents, sont choisis parmi les radicaux hydrocarbonés en $C_7$-$C_{21}$, linéaires ou ramifiés, saturés ou insaturés ;
- $R_{18}$ est choisi parmi :

  - le radical

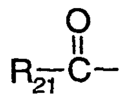

- l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange constitué d'au moins un agent tensioactif anionique et d'au moins un agent tensioactif amphotère.

La composition de l'invention peut également contenir au moins un additif choisi parmi les épaississants, les parfums, les agents nacrants, les conservateurs, les filtres solaires siliconés ou non, les vitamines, les provitamines, les polymères anioniques ou non ioniques, les protéines non cationiques, les hydrolysats de protéines non cationiques, l'acide méthyl-18 eicosanoique, les hydroxyacides, le panthénol, et tout autre additif classiquement utilisé dans le domaine cosmétique qui n'affecte pas les propriétés des compositions selon l'invention.

Ces additifs sont présents dans la composition selon l'invention dans des proportions pouvant aller de 0 à 20% en poids par rapport au poids total de la composition. La quantité précise de chaque additif est déterminée facilement par l'homme du métier selon sa nature et sa fonction.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Lles compositions selon l'invention peuvent être des compositions détergentes telles que des shampooings, des gels-douche et des bains moussants. Dans ce mode de réalisation de l'invention, les compositions comprennent une base lavante, généralement aqueuse.

Le ou les tensioactifs formant la base lavante peuvent être indifféremment choisis, seuls ou en mélanges, au sein des tensioactifs anioniques, amphotères, non ioniques et cationiques tels que définis ci-dessus.

On utilise de préférence un agent tensioactif anionique choisi parmi les alkyl($C_{12}$-$C_{14}$) sulfates de sodium, de triéthanolamine ou d'ammonium, les alkyl ($C_{12}$-$C_{14}$)éthersulfates de sodium, de triéthanolamine ou d'ammonium oxyéthylénés à 2,2 moles d'oxyde d'éthylène, le cocoyl iséthionate de sodium et l'alphaoléfine($C_{14}$-$C_{16}$) sulfonate de sodium et leurs mélange avec :

- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La quantité et la qualité de la base lavante sont celles suffisantes pour conférer à la composition finale un pouvoir moussant et/ou détergent satisfaisant.

Ainsi, selon l'invention, la base lavante peut représenter de 4 % à 50 % en poids, de préférence de ,6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre

les deux étapes d'une permanente ou d'un défrisage.

Lorsque la composition se présente sous la forme d'un après-shampooing éventuellement à rincer, elle contient avantageusement au moins un tensioactif cationique, sa concentration généralement comprise entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

Dans les exemples, MA signifie matière active.

Dans les exemples, les noms commerciaux ont les définitions suivantes :

EXEMPLE 1

On a réalisé un après-shampooing conforme à l'invention de composition suivante :

- Inuline (RAFTILINE HP de ORAFTI) 3 g MA
- Chlorure de béhényl triméthyl ammonium 1,8 g MA
- Chlorure de Méthacrylate d'éthyl triméthyl ammonium (SALCARE SC 95 de CIBA) 1,5 g MA
- Amodimethicone (BELSIL ADM 6057 E de WACKER) 1,7 gMA
- Eau qsp 100 g

La composition a une texture épaisse et très fondante lors de l'application sur des cheveux humides. Sa rinçabilité est bonne. Les cheveux mouillés ne sont pas chargés et la mise en forme est aisée.

EXEMPLE 2

On a réalisé un après-shampooing conforme à l'invention de composition suivante :

- Inuline (RAFTILINE HP de ORAFTI) 5 g MA
- Chlorure de Méthacrylate d'éthyl triméthyl ammonium (SALCARE SC 95 de ORAFTI) 1 g MA
- Eau qsp 100 g

Les cheveux traités ont les mêmes propriétés que ceux traités avec la composition de l'exemple 1.

EXEMPLE 3

On a réalisé un après-shampooing conforme à l'invention de composition suivante :

- Inuline (RAFTILINE HP de ORAFTI) 1,7 g MA
- Chlorure de palmitylamidopropyl triméthyl ammonium 1,8 g MA
- Chlorure de Méthacrylate d'éthyl triméthyl ammonium (SALCARE SC 95 de CIBA) 2,3 g MA
- Huile d'avocat 2,5 g
- Eau qsp 100 g

Les cheveux traités ont les mêmes propriétés que ceux traités avec la composition de l'exemple 1.

**EXEMPLE 4**

On a réalisé un après-shampooing conforme à l'invention de composition suivante :

- Inuline (RAFTILINE HP de ORAFTI) 15 g MA
- Chlorure de palmitylamidopropyl triméthyl ammonium 4 g MA
- Homopolymère de chlorure de méthacrylate d'éthyl triméthyl ammonium (SALCARE SC 95 de CIBA) 0,1 g MA
- Myristate d'isopropyle 2 g
- Eau qsp 100 g

Les cheveux traités ont les mêmes propriétés que ceux traités avec la composition de l'exemple 1.

**EXEMPLE 5**

On a réalisé un après-shampooing conforme à l'invention de composition suivante :

- Inuline (RAFTILINE HP de ORAFTI) 8 g MA
- Chlorure de béhényl triméthyl ammonium 2,5 g MA
- Copolymère d' acrylamide et de chlorure de bétamethacrylyloxyethyl trimethyl ammonium (ROHAGIT KF 720 de ROHM) 0,8 g MA
- Divinyldimethicone /diméthicone réticulé en émulsion cationique (DC2-1997 de DOW CORNING) 1 gMA
- Eau qsp 100 g

Les cheveux traités ont les mêmes propriétés que ceux traités avec la composition de l'exemple 1.

EXEMPLE 6

On a réalisé un après-shampooing conforme à l'invention de composition suivante :

- Inuline (RAFTILINE HP de ORAFTI) 15 g MA
- Lauryléthersulfate de sodium à 2,2 moles d'oxyde d'éthylène 1 g MA
- polyquaternium-10 (JR 400 de AMERCHOL) 1,2 g MA
- Huile d'avocat 8 g
- Eau qsp 100g

Les cheveux traités ont les mêmes propriétés que ceux traités avec la composition de l'exemple 1.

**Revendications**

1. Composition cosmétique, **caractérisée par le fait qu'**elle comprend, dans un milieu cosmétiquement acceptable, au moins un polymère cationique et au moins un fructane, le fructane ne comportant pas de groupement amine.

2. Composition selon la revendication 1, **caractérisée par le fait que** le fructane est choisi parmi les inulines.

3. Composition selon l'une des revendications 1 ou 2, **caractérisée par le fait que** les polymères cationiques sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

4. Composition selon l'une quelconque des revendications précédentes, **caractérisée par le fait que** ledit polymère cationique est choisi parmi :

   (1) les homopolymères ou copolymères dérivés d'esters ou d'amides acryliques ou méthacryliques et comportant au moins un des motifs de formules suivantes:

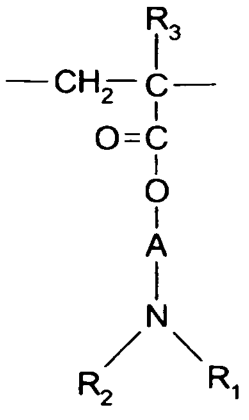
,
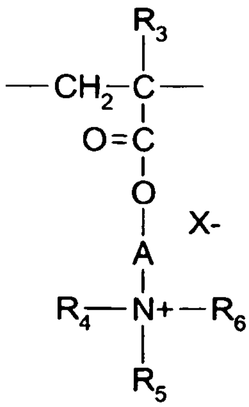

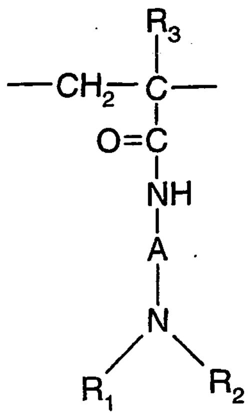

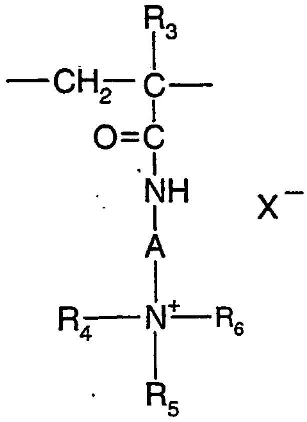

dans lesquelles:

- $R_3$, identiques ou différents, désignent un atome d'hydrogène ou un radical $CH_3$;
- A, identiques ou différents, représentent un groupe alkyle, linéaire ou ramifié, de 1 à 6 atomes de carbone ou un groupe hydroxyalkyle de 1 à 4 atomes de carbone ;
- $R_4$, $R_5$, $R_6$, identiques ou différents, représentent un groupe alkyle ayant de 1 à 18 atomes de carbone ou un radical benzyle;
- $R_1$ et $R_2$ , identiques ou différents, représentent hydrogène ou un groupe alkyle ayant de 1 à 6 atomes de carbone;
- X désigne un anion dérivé d'un acide minéral ou organique.

(2) Les dérivés d'éthers de cellulose comportant des groupements ammonium quaternaires,
(3) Les dérivés de cellulose cationiques tels que les copolymères de cellulose ou les dérivés de cellulose greffés avec un monomère hydrosoluble d'ammonium quaternaire,
(4) Les polysaccharides cationiques,
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères,
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une

bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées.

(7) les dérivés de polyaminoamides résultant de la condensation de polyalcoylènes polyamines avec des acides polycarboxyliques suivie d'une alcoylation par des agents bifonctionnels,

(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone,

(9) les cyclopolymères d'alkyl diallyl amine ou de dialkyl diallyl ammonium

(10) les polymères de diammonium quaternaire contenant des motifs récurrents répondant à la formule :

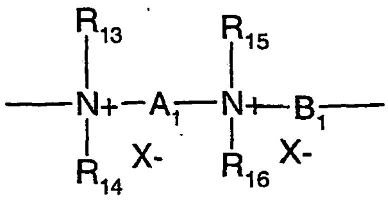

(VII)

formule (VII) dans laquelle :

R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;

A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et

X désigne un anion dérivé d'un acide minéral ou organique;

A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-

dans lequel D désigne :

a) un reste de glycol de formule : -O-Z-O-, où Z désigne un radical hydrocarboné linéaire ou ramifié ou un groupement répondant à l'une des formules suivantes :

-(CH2-CH2-O)x-CH2-CH2-

-[CH2-CH(CH3)-O]y-CH2-CH(CH3)-

où x et y désignent un nombre entier de 1 à 4, représentant un degré de polymérisation défini et unique ou un nombre quelconque de 1 à 4 représentant un degré de polymérisation moyen ;

b) un reste de diamine bis-secondaire tel qu'un dérivé de pipérazine ;

c) un reste de diamine bis-primaire de formule : -NH-Y-NH-, où Y désigne un radical hydrocarboné linéaire ou ramifié, ou bien le radical bivalent

-CH2-CH2-S-S-CH2-CH2- ;

d) un groupement uréylène de formule : -NH-CO-NH- ;

De préférence, $X^-$ est un anion.

(11) les polymères de polyammonium quaternaires constitués de motifs de formule (VIII):

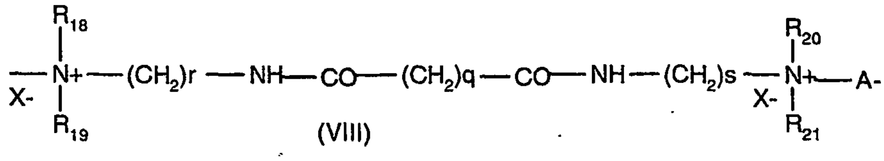

(VIII)

formule dans laquelle :

R18, R19, R20 et R21, identiques ou différents, représentent un atome d'hydrogène ou un radical méthyle, éthyle, propyle, β-hydroxyéthyle, β-hydroxypropyle ou -CH2CH2(OCH2CH2)pOH,
où p est égal à 0 ou à un nombre entier compris entre 1 et 6, sous réserve que R18, R19, R20 et R21 ne représentent pas simultanément un atome d'hydrogène,
r et s, identiques ou différents, sont des nombres entiers compris entre 1 et 6,
q est égal à 0 ou à un nombre entier compris entre 1 et 34,
X désigne un atome d'halogène,
A désigne un radical d'un dihalogénure ou représente de préférence -CH2-CH2-O-CH2-CH2-.

(12) Les polymères quaternaires de vinylpyrrolidone et de vinylimidazole,
(13) Les polyamines référencées sous le nom de « POLYETHYLENEGLYCOL (15) TALLOW POLYAMINE » dans le dictionnaire CTFA.
(14) Les polymères réticulés de sels de méthacryloyloxyalkyl($C_1$-$C_4$) trialkyl($C_1$ -$C_4$)ammonium,
(15) des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

**5.** Composition selon la revendication précédente, **caractérisée par le fait que** ledit polymère cationique est choisi parmi les dérivés d'éther de cellulose quaternaires, les cyclopolymères cationiques, les polysaccharides cationiques, les polymères quaternaires de vinylpyrrolidone et de vinylimidazole, les homopolymères ou copolymères réticulés de sels de méthacryloyloxyalkyl($C_1$-$C_4$) trialkyl($C_1$-$C_4$)ammonium et leurs mélanges.

**6.** Composition selon la revendication 5, **caractérisée par le fait que** ledit cyclopolymère est choisi parmi les homopolymères du chlorure de diallyldiméthylammonium et les copolymères du chlorure de diallyldiméthylammonium et d'acrylamide.

**7.** Composition selon la revendication 5, **caractérisée par le fait que** lesdits dérivés d'éther de cellulose quaternaires sont choisis parmi les hydroxyéthylcelluloses ayant réagi avec un époxyde substitué par un groupement triméthylammonium.

**8.** Composition selon la revendication 5, **caractérisée par le fait que** lesdits polysaccharides cationiques sont choisis parmi les gommes de guar modifiées par un sel de 2,3-époxypropyl triméthylammonium.

**9.** Composition selon l'une quelconque des revendications 1 à 8, **caractérisée par le fait qu'**elle comprend en outre au moins une silicone.

**10.** Composition selon la revendication 9, **caractérisée par le fait que** les silicones sont choisies parmi les polyorganosiloxanes insolubles dans la composition.

**11.** Composition selon l'une quelconque des revendications 9 ou 10, **caractérisée par le fait que** les silicones sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

**12.** Composition selon la revendication 11, **caractérisée par le fait que** :

(a) les polyalkylsiloxanes sont choisis parmi :

- les polydiméthylsiloxanes à groupements terminaux triméthylsilyle ;
- les polydiméthylsiloxanes à groupements terminaux diméthylsilanol ;
- les polyalkyl($C_1$-$C_{20}$)siloxanes ;

(b) les polyalkylarylsiloxanes sont choisis parmi :

- les polydiméthylméthylphénylsiloxanes, les polydiméthyldiphényl siloxanes linéaires et/ou ramifiés de viscosité comprise entre $1.10^{-5}$ et $5.10^{-2} m^2/s$ à 25°C ;

(c) les gommes de silicone sont choisies parmi les polydiorganosiloxanes ayant des masses moléculaires moyennes en nombre comprises entre 200 000 et 1 000 000 utilisés seuls ou sous forme de mélange dans un solvant ;
(d) les résines sont choisies parmi les résines constituées d'unités : $R_3$ Si $O_{1/2}$, $R_2$ Si $O_{2/2}$, R Si $O_{3/2}$, Si $O_{4/2}$ dans lesquelles R représente un groupement hydrocarboné ayant de 1 à 16 atomes de carbone ou un groupement phényle ;
(e) les silicones organo modifiées sont choisies parmi les silicones comportant dans leur structure un ou plusieurs groupements organofonctionnels fixés par l'intermédiaire d'un radical hydrocarboné.

**13.** Composition selon l'une quelconque des revendications 9 à 12, **caractérisée par le fait que** les silicones sont choisies parmi les polyaikylsiloxanes à groupements terminaux triméthylsilyle, les polyalkylsiloxanes à groupements terminaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes.

**14.** Composition selon l'une quelconque des revendications 1 à 13, **caractérisée par le fait qu'**elle comprend en outre au moins un agent bénéfique pour les cheveux choisi parmi les esters d'acides carboxyliques en C1-C30 et de d'alcools mono ou polyhydroxylés en C1-C30, les huiles végétales, animales, minérales ou de synthèse, les cires, les céramides, les pseudocéramides..

**15.** Composition selon l'une quelconque des revendications précédentes, **caractérisée en ce que** le fructane est présent à une concentration comprise entre 0,01 % et 20 % en poids par rapport au poids total de la composition, de préférence entre 0,05 % et 15 % en poids.

**16.** Composition selon l'une quelconque des revendications précédentes, **caractérisée en ce que** le polymère cationique est présent à une concentration comprise entre 0,001 % et 20 % en poids par rapport au poids total de la composition, de préférence entre 0,01 % et 10 % en poids.

**17.** Composition selon l'une quelconque des revendications 9 à 16, **caractérisée en ce que** ladite silicone est présente à une concentration comprise entre 0,001 % et 20 % en poids par rapport au poids total de la composition, de préférence entre 0,01 % et 10 % en poids.

**18.** Composition selon l'une quelconque des revendications 14 à 17, **caractérisée en ce que** l'agent bénéfique pour les cheveux est présent à une concentration comprise entre 0,001 % et 20 % en poids par rapport au poids total de la composition, de préférence entre 0,01 % et 10 % en poids.

**19.** Composition selon l'une quelconque des revendications précédentes, **caractérisée par le fait qu'**elle comprend en outre au moins un agent tensioactif choisi parmi les tensioactifs anioniques, non ioniques, amphotères, cationiques et leurs mélanges.

**20.** Composition selon la revendication 19, **caractérisée par le fait que** le ou les agents tensioactifs sont présents à une concentration comprise entre 0,01% et 50% en poids, de préférence entre 0,1% et 40% en poids, et encore

plus préférentiellement entre 0,5% et 30% en poids, par rapport au poids total de la composition.

**21.** Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

**22.** Utilisation d'une composition telle que définie dans l'une quelconque des revendications précédentes pour le lavage ou pour le soin des matières kératiniques.

**23.** Procédé de traitement des matières kératiniques, telles que les cheveux, **caractérisé en ce qu'**il consiste à appliquer sur lesdites matières une composition cosmétique selon l'une des revendications 1 à 21, puis à effectuer éventuellement un rinçage à l'eau.

**24.** Utilisation d'un fructane tel que défini à l'une des revendications 1 ou 2 dans, ou pour la fabrication d'une composition cosmétique comprenant un polymère cationique.

## Claims

**1.** Cosmetic composition, **characterized in that** it comprises, in a cosmetically acceptable medium, at least one cationic polymer and at least one fructan, the fructan not comprising an amine group.

**2.** Composition according to Claim 1, **characterized in that** the fructan is chosen from inulins.

**3.** Composition according to either of Claims 1 and 2, **characterized in that** the cationic polymers are chosen from those which contain units comprising primary, secondary, tertiary and/or quaternary amine groups which can either be part of the main polymer chain, or be carried by a side substituent directly linked to said chain.

**4.** Composition according to any one of the preceding claims, **characterized in that** said cationic polymer is chosen from:

(1) homopolymers or copolymers derived from acrylic or methacrylic esters or amides comprising at least one of the units of the following formulae:

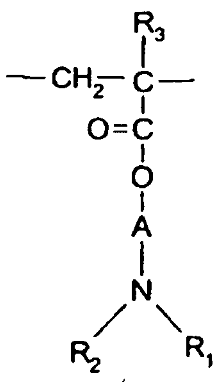,

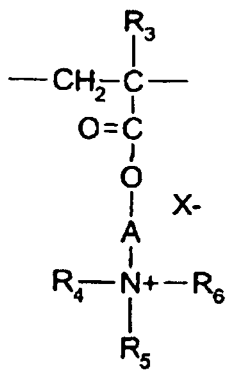

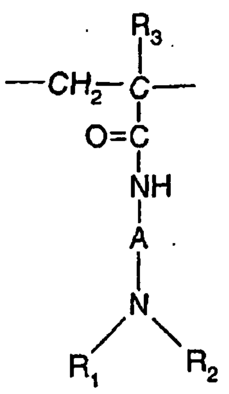

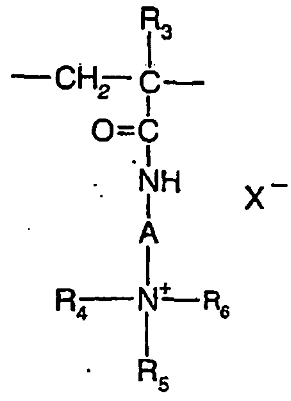

in which:

$R_3$, which may be identical or different, denote a hydrogen atom or a $CH_3$ radical;
A, which may be identical or different, represent a linear or branched alkyl group having from 1 to 6 carbon atoms or a hydroxyalkyl group having from 1 to 4 carbon atoms;
$R_4$, $R_5$ and $R_6$, which may be identical or different, represent an alkyl group having from 1 to 18 carbon atoms or a benzyl radical;
$R_1$ and $R_2$, which may be identical or different, represent hydrogen or an alkyl group having from 1 to 6 carbon atoms;
X denotes an anion derived from an inorganic or organic acid,

(2) derivatives of cellulose ethers comprising quaternary ammonium groups,
(3) cationic cellulose derivatives such as cellulose copolymers or cellulose derivatives grafted with a water-soluble quaternary ammonium monomer,
(4) cationic polysaccharides,
(5) polymers consisting of piperazinyl units and of divalent, straight- or branched-chain alkylene or hydroxyalkylene radicals, optionally interrupted with oxygen, sulphur or nitrogen atoms or with aromatic or heterocyclic rings, and also the oxidation and/or quaternization products of these polymers,
(6) water-soluble polyaminoamides prepared, in particular, by polycondensation of an acidic compound with a polyamine; these polyaminoamides can be crosslinked with an epihalohydrin, a diepoxide, a dianhydride, an unsaturated dianhydride, a bisunsaturated derivative, a bishalohydrin, a bisazetidinium, a bishaloacyldiamine or an alkyl bishalide, or alternatively with an oligomer resulting from the reaction of a bifunctional compound reactive with respect to a bishalohydrin, a bisazetidinium, a bishaloacyldiamine, an alkyl bishalide, an epihalohydrin, a diepoxide or a bisunsaturated derivative; the crosslinking agent being used in proportions ranging from 0.025 to 0.35 mol per amine group of the polyaminoamide; these polyaminoamides can be alkylated or, if they comprise one or more tertiary amine functions, quaternized,
(7) polyaminoamide derivatives resulting from the condensation of polyalkylenepolyamines with polycarboxylic acids, followed by alkylation with bifunctional agents,
(8) polymers obtained by reaction of a polyalkylenepolyamine comprising two primary amine groups and at least one secondary amine group with a dicarboxylic acid chosen from diglycolic acid and saturated aliphatic dicarboxylic acids having from 3 to 8 carbon atoms,
(9) cyclopolymers of alkyldiallylamine or of dialkyldiallylammonium,
(10) quaternary diammonium polymers comprising repeat units corresponding to the formula:

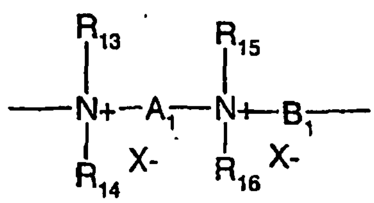 (VII)

in which formula (VII):

$R_{13}$, $R_{14}$, $R_{15}$ and $R_{16}$, which may be identical or different, represent aliphatic, alicyclic or arylaliphatic radicals containing from 1 to 20 carbon atoms or lower aliphatic hydroxyalkyl radicals, or else $R_{13}$, $R_{14}$, $R_{15}$ and $R_{16}$, together or separately, form, with the nitrogen atoms to which they are attached, heterocycles optionally comprising a second heteroatom other than nitrogen, or else $R_{13}$, $R_{14}$, $R_{15}$ and $R_{16}$ represent a linear or branched $C_1$-$C_6$ alkyl radical substituted with a nitrile, ester, acyl, amide or -CO-O-$R_{17}$-D or -CO-NH-$R_{17}$-D group, where $R_{17}$ is an alkylene and D a quaternary ammonium group;
$A_1$ and $B_1$ represent polymethylene groups containing from 2 to 20 carbon atoms which can be linear or branched and saturated or unsaturated, and which can comprise, bonded to or inserted into the main chain, one or more aromatic rings or one or more oxygen or sulphur atoms or sulphoxide, sulphone, disulphide, amino, alkylamino, hydroxyl, quaternary ammonium, ureido, amide or ester groups, and
$X^-$ denotes an anion derived from an inorganic or organic acid;
$A_1$, $R_{13}$ and $R_{15}$ can form, with the two nitrogen atoms to which they are attached, a piperazine ring; in addition, if $A_1$ denotes a linear or branched and saturated or unsaturated alkylene or hydroxyalkylene radical, $B_1$ can also denote a $(CH_2)_n$-CO-D-OC-$(CH_2)_n$-group in which D denotes:

a) a glycol residue of formula: -O-Z-O, where Z denotes a linear or branched hydrocarbon-based radical or a group corresponding to one of the following formulae:

$$-(CH_2-CH_2-O)_x-CH_2-CH_2-$$

$$-[CH_2-CH(CH_3)-O]y-CH_2-CH(CH_3)-$$

where x and y denote an integer from 1 to 4, representing a defined and unique degree of polymerization, or any number from 1 to 4 representing a mean degree of polymerization;
b) a bissecondary diamine residue, such as a piperazine derivative;
c) a bisprimary diamine residue of formula: -NH-Y-NH-, where Y denotes a linear or branched hydrocarbon-based radical or else the divalent radical

$$-CH_2-CH_2-S-S-CH_2-CH_2- ;$$

d) a ureylene group of formula: -NH-CO-NH-;

preferably, X- is an anion,

(11) poly(quaternary ammonium) polymers consisting of units of formula (VIII):

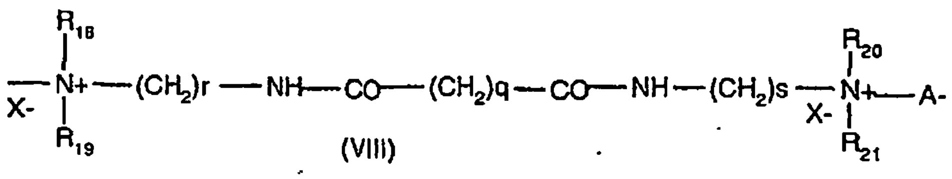

(VIII)

in which formula:

$R_{18}$, $R_{19}$, $R_{20}$ and $R_{21}$, which may be identical or different, represent a hydrogen atom or a methyl, ethyl, propyl, β-hydroxyethyl, β-hydroxypropyl or $-CH_2CH_2(OCH_2CH_2)pOH$ radical,
where p is equal to 0 or to an integer between 1 and 6, with the proviso that $R_{18}$, $R_{19}$, $R_{20}$ and $R_{21}$ do not simultaneously represent a hydrogen atom,
r and s, which may be identical or different, are integers between 1 and 6,
q is equal to 0 or to an integer between 1 and 34,
X denotes a halogen atom,
A denotes a radical of a dihalide, or preferably represents $-CH_2-CH_2-O-CH_2-CH_2-$,

(12) quaternary polymers of vinylpyrrolidone and of vinylimidazole,
(13) polyamines referenced under the name "Polyethylene Glycol (15) Tallow Polyamine" in the CTFA dictionary,
(14) crosslinked polymers of methacryloyloxy($C_1$-$C_4$)-alkyltri ($C_1$-$C_4$) alkylammonium salts,
(15) polyalkyleneimines, in particular polyethyleneimines, polymers comprising vinylpyridine or vinylpyridinium units, condensates of polyamines and of epichlorohydrin, quaternary polyureylenes and chitin derivatives.

**5.** Composition according to the preceding claim, **characterized in that** said cationic polymer is chosen from quaternary cellulose ether derivatives, cationic cyclopolymers, cationic polysaccharides, quaternary polymers of vinylpyrrolidone and of vinylimidazole, crosslinked homopolymers or copolymers of methacryloyloxy ($C_1$-$C_4$) alkyltri ($C_1$-$C_4$) alkylammonium salts, and mixtures thereof.

**6.** Composition according to Claim 5, **characterized in that** said cyclopolymer is chosen from homopolymers of diallyldimethylammonium chloride and copolymers of diallyldimethylammonium chloride and of acrylamide.

**7.** Composition according to Claim 5, **characterized in that** said quaternary cellulose ether derivatives are chosen from hydroxyethylcelluloses which have reacted with an epoxide substituted with a trimethylammonium group.

**8.** Composition according to Claim 5, **characterized in that** said cationic polysaccharides are chosen from guar gums modified with a 2,3-epoxypropyltrimethylammonium salt.

**9.** Composition according to any one of Claims 1 to 8, **characterized in that** it also comprises at least one silicone.

**10.** Composition according to Claim 9, **characterized in that** the silicones are chosen from polyorganosiloxanes insoluble in the composition.

**11.** Composition according to either one of Claims 9 and 10, **characterized in that** the silicones are nonvolatile polyorganosiloxanes chosen from polyalkylsiloxanes, polyarylsiloxanes, polyalkylarylsiloxanes, silicone gums and resins, polyorganosiloxanes modified with organofunctional groups, and mixtures thereof.

**12.** Composition according to Claim 11, **characterized in that**:

(a) the polyalkylsiloxanes are chosen from:

- polydimethylsiloxanes with trimethylsilyl end groups;
- polydimethylsiloxanes with dimethylsilanol end groups;
- poly ($C_1$-$C_{20}$) alkylsiloxanes ;

(b) the polyalkylarylsiloxanes are chosen from:

- polydimethylmethylphenylsiloxanes or polydimethyldiphenylsiloxanes which are linear and/or branched, and have a viscosity of between 1 x $10^{-5}$ and 5 x $10^{-2}$ $m^2/s$ at 25°C;

(c) the silicone gums are chosen from polydiorganosiloxanes having number-average molecular masses of between 200 000 and 1 000 000, used alone or in the form of a mixture in a solvent;
(d) the resins are chosen from resins consisting of units: $R_3SiO_{1/2}$, $R_2SiO_{2/2}$, $RSiO_{3/2}$, $SiO_{4/2}$,
in which R represents a hydrocarbon-based group having from 1 to 16 carbon atoms or a phenyl group;
(e) the modified organosilicones are chosen from silicones comprising, in their structure, one or more organofunctional groups attached by means of a hydrocarbon-based radical.

**13.** Composition according to any one of Claims 9 to 12, **characterized in that** the silicones are chosen from polyalkylsiloxanes with trimethylsilyl end groups, polyalkylsiloxanes with dimethylsilanol end groups, polyalkylarylsiloxanes, mixtures of two PDMSs consisting of a gum and of an oil of different viscosities, mixtures of cyclic silicones and organosiloxanes, and organopolysiloxane resins.

**14.** Composition according to any one of Claims 1 to 13, **characterized in that** it also comprises at least one agent which is beneficial to the hair, chosen from esters of $C_1$-$C_{30}$ carboxylic acids and of $C_1$-$C_{30}$ mono- or polyhydroxylated alcohols, plant, animal, mineral or synthetic oils, waxes, ceramides, pseudoceramides, etc.

**15.** Composition according to any one of the preceding claims, **characterized in that** the fructan is present at a concentration of between 0.01% and 20% by weight relative to the total weight of the composition, preferably of between 0.05% and 15% by weight.

**16.** Composition according to any one of the preceding claims, **characterized in that** the cationic polymer is present at a concentration of between 0.001% and 20% by weight relative to the total weight of the composition, preferably of between 0.01% and 10% by weight.

**17.** Composition according to any one of Claims 9 to 16, **characterized in that** said silicone is present at a concentration of between 0.001% and 20% by weight relative to the total weight of the composition, preferably of between 0.01% and 10% by weight.

**18.** Composition according to any one of Claims 14 to 17, **characterized in that** the agent beneficial to the hair is present at a concentration of between 0.001% and 20% by weight relative to the total weight of the composition, preferably of between 0.01% and 10% by weight.

**19.** Composition according to any one of the preceding claims, **characterized in that** it also comprises at least one surfactant chosen from anionic, non-ionic, amphoteric and cationic surfactants, and mixtures thereof.

**20.** Composition according to Claim 19, **characterized in that** the surfactant(s) is (are) present at a concentration of between 0.01% and 50% by weight, preferably of between 0.1% and 40% by weight, and even more preferentially of between 0.5% and 30% by weight, relative to the total weight of the composition.

**21.** Composition according to any one of the preceding claims, **characterized in that** it is in the form of a shampoo, a conditioner, a composition for permanent-waving, relaxing, dyeing or bleaching the hair, a rinse-out composition to be applied between the two steps of a permanent-waving or hair-relaxing operation, or a washing composition for the body.

**22.** Use of a composition as defined in any one of the preceding claims, for washing or for caring for keratin materials.

**23.** Process for treating keratin materials, such as the hair, **characterized in that** it consists in applying a cosmetic composition according to one of Claims 1 to 21 to said materials, and then optionally rinsing with water.

**24.** Use of a fructan as defined in either of Claims 1 and 2, in or for the manufacture of a cosmetic composition comprising a cationic polymer.

**Patentansprüche**

**1.** Kosmetische Zusammensetzung, **dadurch gekennzeichnet, dass** sie in einem kosmetisch akzeptablen Medium mindestens ein kationisches Polymer und mindestens ein Fructan enthält, wobei das Fructan keine Aminogruppe aufweist.

**2.** Zusammensetzung nach Anspruch 1, **dadurch gekennzeichnet, dass** das Fructan unter den Inulinen ausgewählt ist.

**3.** Zusammensetzung nach Anspruch 1 oder 2, **dadurch gekennzeichnet, dass** die kationischen Polymere unter den Polymeren ausgewählt sind, die als Teil der Polymerhauptkette oder an einem direkt an die Hauptkette gebundenen seitlichen Substituenten primäre, sekundäre, tertiäre und/oder quartäre Aminogruppen enthalten.

**4.** Zusammensetzung nach einem der vorhergehenden Ansprüche, **dadurch gekennzeichnet, dass** das kationische Polymer ausgewählt ist unter:

(1) Homopolymeren oder Copolymeren, die von Acrylsäureestern, Methacrylsäureestern, Acrylamiden oder Methacrylamiden abgeleitet sind und die mindestens eine Einheit der folgenden Formeln enthalten:

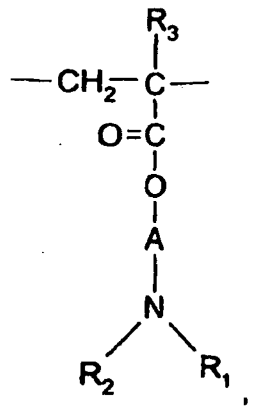

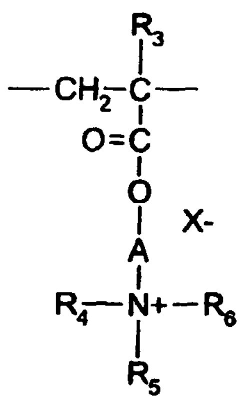

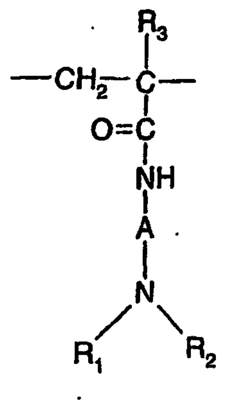

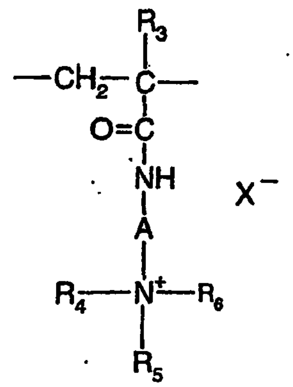

worin bedeuten:

die Gruppen $R_3$, die gleich oder verschieden sind, ein Wasserstoffatom oder die Gruppe $CH_3$ ;
die Gruppen A, die gleich oder verschieden sind, eine geradkettige oder verzweigte Alkylgruppe mit 1 bis 6 Kohlenstoffatomen oder eine Hydroxyalkylgruppe mit 1 bis 4 Kohlenstoffatomen;
die Gruppen $R_4$, $R_5$ und $R_6$, die gleich oder verschieden sind, eine Alkylgruppe mit 1 bis 18 Kohlenstoffatomen oder eine Benzylgruppe;
die Gruppen $R_1$ und $R_2$, gleich oder verschieden sind, Wasserstoff oder eine Alkylgruppe mit 1 bis 6 Kohlenstoffatomen;
X ein von einer anorganischern oder organischen Säure abgeleitetes Anion;

(2) Celluloseetherderivaten, die quartäre Ammoniumgruppen enthalten;
(3) kationischen Cellulosederivaten, wie Cellulose-Copolymeren oder Cellulose-Derivaten, die mit einem wasserlöslichen quartären Ammoniummonomer gepfropft sind;
(4) kationischen Polysacchariden;
(5) Polymeren, die aus Piperazinyleinheiten und zweiwertigen, geraden oder verzweigten Alkylen- oder Hydroxyalkylengruppen bestehen, die gegebenenfalls durch Sauerstoffatome, Schwefelatome, Stickstoffatome oder aromatische oder heterocyclische Ringe unterbrochen sind, sowie den Oxidationsprodukten und/ oder Quaternisierungsprodukten dieser Polymere;
(6) Wasserlöslichen Polyaminoamiden, die insbesondere durch Polykondensation einer Säure mit einem Polyamin hergestellt sind; diese Polyaminoamide können mit einem Epihalohydrin, einem Diepoxid, einem Dianhydrid, einem ungesättigten Dianhydrid, einem zweifach ungesättigten Derivat, einem Bis-halohydrin, einem Bis-azetidinium, einem Bis-haloacyldiamin, einem Alkyl-bis-halogenid oder auch einem Oligomer vernetzt sein, das bei der Umsetzung einer bifunktionellen Verbindung entsteht, die gegenüber einem Bis-halohydrin, Bis-azetidinium, Bis-haloacyldiamin, Alkyl-bis-halogenid, Epihalohydrin, Diepoxid oder zweifach ungesättigten Derivat reaktiv ist; das Vernetzungsmittel wird in Mengenanteilen von 0,025 bis 0,35 Mol pro Aminogruppe des Polyaminoamids eingesetzt. Die Polyaminoamide können alkyliert oder, wenn sie eine oder mehrere tertiäre

Aminogruppen aufweisen, quaternisiert sein;

(7) Derivaten von Polyaminoamiden, die bei der Kondensation von Polyalkylenpolyaminen mit Polycarbonsäuren und anschließender Alkylierung mit bifunktionellen Mitteln entstehen;

(8) Polymeren, die durch Umsetzung eines Polyalkylenpolyamins, das zwei primäre Aminogruppen und mindestens eine sekundäre Aminogruppe aufweist, mit einer Dicarbonsäure erhalten werden, die unter Glycolsäure und den gesättigten aliphatischen Dicarbonsäuren mit 3 bis 8 Kohlenstoffatomen ausgewählt ist;

(9) Alkyldiallylamin-Cyclopolymeren oder Dialkyldiallylammonium-Cyclopolymeren;

(10) Quartären Diammoniumpolymeren mit wiederkehrenden Einheiten der folgenden Formel:

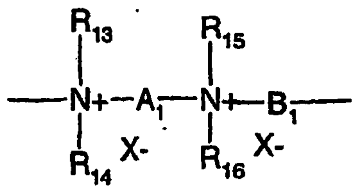

(VII)

wobei in der Formel (VII) bedeuten:

die Gruppen $R_{13}$, $R_{14}$, $R_{15}$ und $R_{16}$, die identisch oder voneinander verschieden sind, bedeuten aliphatische, alicyclische oder arylaliphatische Gruppen mit 1 bis 20 Kohlenstoffatomen oder niedere hydroxyalkylaliphatische Gruppen oder die Gruppen $R_{13}$, $R_{14}$, $R_{15}$ und $R_{16}$ bilden gemeinsam oder unabhängig voneinander mit den Stickstoffatomen, an die sie gebunden sind, Heterocyclen, die gegebenenfalls ein zweites Heteroatom enthalten, das von Stickstoff verschieden ist, oder die Gruppen $R_{13}$, $R_{14}$, $R_{15}$ und $R_{16}$ bedeuten eine geradkettige oder verzweigte $C_{1-6}$-Alkylgruppe, die mit einer Nitril-, Ester-, Acyl- oder Amidgruppe oder -CO-O-$R_{17}$-D oder -CO-NH-$R_{17}$-D substituiert ist, worin $R_{17}$ eine Alkylengruppe und D eine quartäre Ammoniumgruppe bedeutet;

$A_1$ und $B_1$ bedeuten Polymethylengruppen mit 2 bis 20 Kohlenstoffatomen, die geradkettig oder verzweigt, gesättigt oder ungesättigt sein können und die an die Hauptkette gebunden oder in der Hauptkette einen oder mehrere aromatische Ringe oder ein oder mehrere Sauerstoffatome oder Schwefelatome oder eine oder mehrere der folgenden Gruppen enthalten können: Sulfoxid, Sulfon, Disulfid, Amino, Alkylamino, Hydroxy, quartäres Ammonium, Ureido, Amid oder Ester, und

X- bedeutet ein Anion, das von einer anorganischen oder organischen Säure abgeleitet ist;

die Gruppen $A_1$, $R_{13}$ und $R_{15}$ können mit den beiden Stickstoffatomen, an die sie gebunden sind, einen Piperazinring bilden; wenn $A_1$ eine geradkettige oder verzweigte, gesättigte oder ungesättigte Alkylen- oder Hydroxyalkylengruppe bedeutet, kann die Gruppe $B_1$ auch eine Gruppe $-(CH_2)_n$-CO-D-OC-$(CH_2)_n$- sein, worin D bedeutet:

a) einen Glycolrest der Formel: -O-Z-O- , worin Z eine geradkettige oder verzweigte Kohlenwasserstoffgruppe oder eine Gruppe bedeutet, die einer der folgenden Formeln entspricht:

$$-(CH_2-CH_2-O)_x-CH_2-CH_2-$$

$$-[CH_2-CH(CH_3)-O]_y-CH_2-CH(CH_3)-$$

worin x und y eine ganze Zahl von 1 bis 4 bedeuten und einen wohl definierten und einzigen Polymerisationsgrad darstellen, oder irgendeine Zahl von 1 bis 4 bedeuten und einen mittleren Polymerisationsgrad angeben;

b) einen bis-sekundären Diaminrest, beispielsweise ein Piperazinderivat,

c) einen bis-primären Diaminrest der Formel: -NH-Y-NH-, worin Y eine geradkettige oder verzweigte Kohlenwasserstoffgruppe oder auch die zweiwertige Gruppe $-CH_2-CH_2-S-S-CH_2-CH_2-$ bedeutet, oder

d) die Ureylengruppe der Formel: -NH-CO-NH-;

$X^-$ bedeutet vorzugsweise ein Anion;

(11) Quartären Polyammoniumpolymeren, die aus wiederkehrenden Einheiten der Formel (VIII) bestehen:

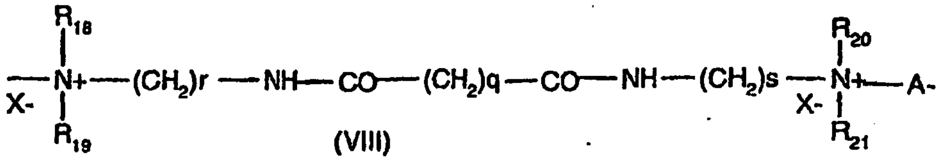

(VIII)

wobei in der Formel bedeuten:

$R_{18}$, $R_{19}$, $R_{20}$ und $R_{21}$, die identisch oder voneinander verschieden sind, ein Wasserstoffatom oder Methyl, Ethyl, Propyl, β-Hydroxyethyl, β-Hydroxypropyl oder $-CH_2CH_2(OCH_2CH_2)pOH$, wobei p Null oder eine ganze Zahl im Bereich von 1 bis 6 bedeutet, mit der Maßgabe, dass $R_{18}$, $R_{19}$, $R_{20}$ und R21 nicht gleichzeitig Wasserstoff bedeuten,
r und s, die gleich oder verschieden sind, eine ganze Zahl im Bereich von 1 bis 6,
q Null oder eine ganze Zahl im Bereich von 1 bis 34,
X ein Halogenatom, und
A ein Dihalogenid oder vorzugsweise $-CH_2-CH_2-O-CH_2-CH_2-$;

(12) Quartären Polymeren von Vinylpyrrolidon und Vinylimidazol;
(13) Polyaminen, die nach CTFA-Nomenklatur als 'POLYETHYLENGLYCOL (15) TALLOW POLYAMINE' bezeichnet werden;
(14) Vernetzten Polymeren von Methacryloyloxyalkyl($C_{1-4}$)trialkyl($C_{1-4}$)ammoniumsalzen;
(15) Polyalkyleniminen, insbesondere Polyethyleniminen, Polymeren, die Vinylpyridin- oder Vinylpyridiniumeinheiten enthalten, Kondensaten von Polyaminen und Epichlorhydrin, quartären Polyureylenen und Chitinderivaten.

**5.** Zusammensetzung nach dem vorhergehenden Anspruch, **dadurch gekennzeichnet, dass** das kationische Polymer unter quartären Celluloseetherderivaten, kationischen Cyclopolymeren, kationischen Polysacchariden, quartären Polymeren von Vinylpyrrolidon und Vinylimidazol, vernetzten Homo- und Copolymeren von Methacryloyloxyalkyl($C_{1-4}$)trialkyl($C_{1-4}$)ammoniumsalzen und deren Gemischen ausgewählt ist.

**6.** Zusammensetzung nach Anspruch 5, **dadurch gekennzeichnet, dass** das Cyclopolymer unter den Homopolymeren von Diallyldimethylammoniumchlorid und den Copolymeren von Diallyldimethylammoniumchlorid und Acrylamid ausgewählt ist.

**7.** Zusammensetzung nach Anspruch 5, **dadurch gekennzeichnet, dass** die quartären Celluloseetherderivate unter den Hydroxyethylcellulosen ausgewählt sind, die mit einem Epoxid, das mit einer Trimethylammoniumgruppe substituiert ist, umgesetzt wurden.

**8.** Zusammensetzung nach Anspruch 5, **dadurch gekennzeichnet, dass** die kationischen Polysaccharide unter den Guargummen ausgewählt sind, die mit einem 2,3-Epoxypropyltrimethylammoniumsalz modifiziert wurden.

**9.** Zusammensetzung nach einem der Ansprüche 1 bis 8, **dadurch gekennzeichnet, dass** sie ferner mindestens ein Silicon enthält.

**10.** Zusammensetzung nach Anspruch 9, **dadurch gekennzeichnet, dass** die Silicone unter den in der Zusammensetzung unlöslichen Polyorganosiloxanen ausgewählt sind.

**11.** Zusammensetzung nach einem der Ansprüche 9 oder 10, **dadurch gekennzeichnet, dass** die Silicone nichtflüchtige Polyorganosiloxane sind, die unter den Polyalkylsiloxanen, Polyarylsiloxanen, Polyalkylarylsiloxanen, Silicongummis und Siliconharzen, mit organofunktionellen Gruppen modifizierten Polyorganosiloxanen sowie deren Gemischen ausgewählt sind.

**12.** Zusammensetzung nach Anspruch 11, **dadurch gekennzeichnet, dass**

(a) die Polyalkylsiloxane unter den folgenden Verbindungen ausgewählt sind:

- den Polydimethylsiloxanen mit endständigen Trimethylsilylgruppen;
- den Polydimethylsiloxanen mit endständigen Dimethylsilanolgruppen;
- den Polyalkyl($C_{1-20}$)siloxanen;

(b) die Polyalkylarylsiloxane unter den folgenden Verbindungen ausgewählt sind:

- den Polydimethylmethylphenylsiloxanen und Polydimethyldiphenylsiloxanen, die geradkettig und/oder verzweigt vorliegen und bei 25°C eine Viskosität von $1 \cdot 10^{-5}$ bis $5.10^{-2}$ $m^2/s$ aufweisen;

(c) die Silicongummis unter den Polydiorganosiloxanen mit einer zahlenmittleren Molmasse im Bereich von 200 000 bis 1 000 000, die als solche oder im Gemisch mit einem Lösungsmittel verwendet werden, ausgewählt sind;
(d) die Harze unter den Harzen ausgewählt sind, die aus Einheiten: $R_3SiO_{1/2}$, $R_2SiO_{2/2}$, $RSiO_{3/2}$ und $SiO_{4/2}$ bestehen, wobei die Gruppe R eine Kohlenwasserstoffgruppe mit 1 bis 16 Kohlenstoffatomen oder eine Phenylgruppe bedeutet;
(e) die organomodifizierten Silicone unter den Siliconen ausgewählt sind, die in ihrer Struktur eine oder mehrere organofunktionelle Gruppen enthalten, die über eine Kohlenwasserstoffgruppe gebunden sind.

**13.** Zusammensetzung nach einem der Ansprüche 9 bis 12, **dadurch gekennzeichnet, dass** die Silicone unter den Polyalkylsiloxanen mit endständigen Trimethylsilylgruppen, den Polyalkylsiloxanen mit Dimethylsilanolendgruppen, den Polyalkylarylsiloxanen, den Gemischen von zwei PDMS, die aus einem Gummi und einem Öl mit unterschiedlichen Viskositäten bestehen, den Gemischen von Organosiloxanen und cyclischen Siliconen und den Organopolysiloxanharzen ausgewählt sind.

**14.** Zusammensetzung nach einem der Ansprüche 1 bis 13, **dadurch gekennzeichnet, dass** sie ferner mindestens einen Stoff enthält, der eine günstige Wirkung auf das Haar hat und unter den Estern von $C_{1-30}$-Carbonsäuren und ein- oder mehrwertigen $C_{1-30}$-Alkoholen, pflanzlichen, tierischen, mineralischen oder synthetischen Ölen, Wachsen, Ceramiden und Pseudoceramiden ausgewählt ist.

**15.** Zusammensetzung nach einem der vorhergehenden Ansprüche, **dadurch gekennzeichnet, dass** das Fructan in einer Konzentration von 0,01 bis 20 Gew.-%, bezogen auf das Gesamtgewicht der Zusammensetzung, und vorzugsweise 0,05 bis 15 Gew.-% enthalten ist.

**16.** Zusammensetzung nach einem der vorhergehenden Ansprüche, **dadurch gekennzeichnet, dass** das kationische Polymer in einer Konzentration von 0,001 bis 20 Gew.-%, bezogen auf das Gesamtgewicht der Zusammensetzung, und vorzugsweise 0,01 bis 10 Gew.-% enthalten ist.

**17.** Zusammensetzung nach einem der Ansprüche 9 bis 16, **dadurch gekennzeichnet, dass** das Silicon in einer Konzentration von 0,001 bis 20 Gew.-%, bezogen auf das Gesamtgewicht der Zusammensetzung, und vorzugsweise 0,01 bis 10 Gew.-% enthalten ist.

**18.** Zusammensetzung nach einem der Ansprüche 14 bis 17, **dadurch gekennzeichnet, dass** der Stoff mit günstiger Wirkung auf das Haar in einer Konzentration von 0,001 bis 20 Gew.-%, bezogen auf das Gesamtgewicht der Zusammensetzung, und vorzugsweise 0,01 bis 10 Gew.-% enthalten ist.

**19.** Zusammensetzung nach einem der vorhergehenden Ansprüche, **dadurch gekennzeichnet, dass** sie ferner mindestens einen grenzflächenaktiven Stoff enthält, der unter den anionischen, nichtionischen, amphoteren, kationischen grenzflächenaktiven Stoffen und deren Gemischen ausgewählt ist.

**20.** Zusammensetzung nach Anspruch 19, **dadurch gekennzeichnet, dass** der grenzflächenaktive Stoff oder die grenzflächenaktiven Stoffe in einer Menge von 0,01 bis 50 Gew.-%, vorzugsweise 0,1 bis 40 Gew.-% und noch bevorzugter 0,5 bis 30 Gew.-%, bezogen auf das Gesamtgewicht der Zusammensetzung, vorliegen.

**21.** Zusammensetzung nach einem der vorhergehenden Ansprüche, **dadurch gekennzeichnet, dass** sie als Haarwaschmittel, Haarpflegemittel, das nach der Haarwäsche angewandt wird, Zusammensetzung für eine Dauerwelle, Entkräuselung, Färbung oder Entfärbung der Haare, Zusammensetzung, die ausgespült wird und zwischen den beiden Schritten einer Dauerwelle oder Entkräuselung aufgetragen wird, oder reinige Zusammensetzung für den Körper vorliegt.

**22.** Verwendung einer Zusammensetzung nach einem der vorhergehenden Ansprüche zur Reinigung oder zur Pflege von Keratinsubstanzen.

**23.** Verfahren zur Behandlung von Keratinsubstanzen, wie Haaren, **dadurch gekennzeichnet, dass** es darin besteht, auf die Keratinsubstanzen eine kosmetische Zusammensetzung nach einem der Ansprüche 1 bis 21 aufzubringen und dann gegebenenfalls mit Wasser zu spülen.

**24.** Verwendung eines Fructans nach einem der Ansprüche 1 oder 2 in einer kosmetischen Zusammensetzung, die ein kationisches Polymer enthält, oder zur Herstellung einer solchen Zusammensetzung.